# EUROPEAN PATENT APPLICATION

(11) **EP 4 467 092 A1**
(43) Date of publication of application: **27.11.2024**
(21) Application number: 24177203.7
(22) Date of filing: 22.05.2024
(51) Int. Cl.: A61B 18/14, A61B 18/00, A61B 5/00, A61B 34/20

(54) **END EFFECTOR HAVING ELONGATED SUPPORT MEMBER WITH CURVED ELECTRODE LANDING REGION**

(30) Priority: 23.05.2023 US 202363503754 P; 22.04.2024 US 202418641750
(71) Applicant: Biosense Webster (Israel) Ltd., 2066717 Yokneam (IL)
(72) Inventor: AGNEW V, William James, Irvine, 92618 (US); THALER, Sean D., Irvine, 92618 (US); VAN NIEKERK, Pieter Emmelius, Irvine, 92618 (US); RODRIGUEZ, Jasson, Irvine, 92618 (US)
(74) Representative: Carpmaels & Ransford LLP

(57) **Abstract**

An end effector of a medical probe, the end effector including a plurality of elongated support members extending along a longitudinal axis of the end effector and the plurality of elongated support members configured to expand from the longitudinal axis. Each of the plurality of elongated support members includes an electrode landing region. The electrode landing region of each of the plurality of elongated support members includes a first surface facing away from the longitudinal axis and at least one extension extending from the first surface and curving toward the longitudinal axis. The first surface of the electrode landing region includes an electrode coupled to the first surface.

## Description

### CROSS REFERENCE TO RELATED APPLICATIONS

The application claims benefit of priority to prior filed U.S. Provisional Patent Application No. 63/503,754 filed May 23, 2023 (Attorney Docket No.: BIO6843USPSP1 - 253757.000371), which is hereby incorporated by reference in full herein.

### FIELD

The present invention generally relates to medical instruments, and more particular to end effectors for medical probes capable of magnetic position sensing and ablation.

### BACKGROUND

Cardiac arrhythmias, such as atrial fibrillation (AF), occur when regions of cardiac tissue abnormally conduct electrical signals to adjacent tissue. This disrupts the normal cardiac cycle and causes asynchronous rhythm. Certain procedures exist for treating arrhythmia, including surgically disrupting the origin of the signals causing the arrhythmia and disrupting the conducting pathway for such signals. By selectively ablating cardiac tissue by application of energy via a catheter, it is sometimes possible to cease or modify the propagation of unwanted electrical signals from one portion of the heart to another.

Many current ablation approaches in the art utilize radiofrequency (RF) electrical energy to heat tissue. RF ablation can have certain risks related to thermal heating which can lead to tissue charring, burning, steam pop, phrenic nerve palsy, pulmonary vein stenosis, and esophageal fistula.

Cryoablation is an alternative approach to RF ablation that generally reduces thermal risks associated with RF ablation. Maneuvering cryoablation devices and selectively applying cryoablation, however, is generally more challenging compared to RF ablation; therefore cryoablation is not viable in certain anatomical geometries which may be reached by electrical ablation devices.

Some ablation approaches use irreversible electroporation (IRE) to ablate cardiac tissue using nonthermal ablation methods. IRE delivers short pulses of high voltage to tissues and generates an unrecoverable permeabilization of cell membranes. Delivery of IRE energy to tissues using multi-electrode catheters was previously proposed in the patent literature. Examples of systems and devices configured for IRE ablation are disclosed in U.S. Patent No. 11,540,877 and U.S. Patent Pub. No. 2021/0169550A1, 2021/0177503A1, and 2021/0186604A1, each of which are incorporated herein by reference and attached in the Appendix of parent Patent Application No. 63/503,754. Further examples of IRE systems are disclosed in 2021/0169567A1, 2021/0169568A1, 2021/0196372A1, each of which are incorporated herein by reference.

Catheters are commonly used for mapping or ablating cardiac tissue. As will be appreciated, knowing positional information of the catheter can help a physician more accurate perform procedures. A magnetic field can be sensed by positioning a conductive coil in the magnetic field and observing electrical current and/or voltage induced in the coil by a change in the magnetic field that is aligned with an axis of the conductive coil. Because electrical current is induced in the conductive coil, such a coil is also referred to as an inductive coil. Relative position of a sensor including one or more inductive coils can be determined in relation to a known magnetic field source by monitoring the induced electric current and/or voltage.

Navigation sensors which include three coil arrangements aligned along three orthogonal axes to determine position and orientation of the navigation sensor in three dimensions within a known induced magnetic field are disclosed for instance in U.S. Patent Numbers 10,330,742 and 10,677,857, each included by reference as if set forth herein and attached in the Appendix of parent Patent Application No. 63/503,754.

Integrating a navigation sensor into a catheter suitable for intravascular and/or intracardiac treatments can involve crafting the sensor by hand, which can result in significant manufacturing time and labor costs. Navigation sensor components can account for a significant percentage of catheter material costs.

Further, as will be appreciated, knowing where and in what orientation the catheter is located can help a physician more accurately place the catheter and ensure proper delivery of the catheter to the targeted tissue. What is needed, therefore, are systems and methods for simplifying circuitry for mapping and ablating cardiac tissue including circuitry for determining the orientation of the catheter.

### SUMMARY

In some examples, an end effector of a medical probe is disclosed. The end effector can include a plurality of elongated support members extending along a longitudinal axis of the end effector. The plurality of elongated support members can be configured to expand from the longitudinal axis. Each of the plurality of elongated support members can include an electrode landing region. The electrode landing region of each of the plurality of elongated support members can include a first surface facing away from the longitudinal axis. The electrode landing region of each of the plurality of elongated support members can also include at least one extension extending from the first surface and curving toward the longitudinal axis. The first surface of the electrode landing region can also include an electrode coupled to the first surface.

In some examples, the end effector can include an inductive coil coupled to at least one extension.

In some examples, the plurality of elongated support members can be coupled to each other at a proximal end of the end effector and at a distal end of the end effector.

In some examples, the electrode landing region can comprise a contiguous outer surface such that the contiguous outer surface comprises the first surface and a curved outer surface of each extension of the at least one extension and such that a portion of the curved outer surface of each extension of the at least one extension faces the longitudinal axis.

In some examples, the contiguous outer surface can comprise a P-shaped or B-shaped profile.

In some examples, the at least one extension can include further curving to overlap a portion of a second surface of the electrode landing region, the second surface facing toward the longitudinal axis and opposite the first surface. The at least one extension can also include being folded at least 180°.

In some examples, the end effector can further include the at least one extension having a pair of extensions symmetrically extending on either side of the electrode landing region. The end effector can also include the at least one extension being folded such that an end of the at least on extension approaches a second surface of the electrode landing region, the second surface facing toward the longitudinal axis and opposite the first surface.

In some examples, the end effector can also include the at least one extension forming a pocket across a portion of a second surface of the electrode landing region, the second surface facing toward the longitudinal axis and opposite the first surface. The end effector can also include an electrical component having a portion positioned within the pocket. The electrical component can also include an inductive coil such that a portion of the inductive coil is positioned within the pocket. The inductive coil can also comprise an insulated wire strand wound about an axis of the inductive coil. The axis of the inductive coil can include being parallel to the longitudinal axis. The axis of the inductive coil can include being orthogonal to the first surface.

In some examples, the end effector can include further having an inductive coil coupled to the at least one extension such that the inductive coil spirals about a sensor axis orthogonal to the longitudinal axis and parallel to the first surface.

In some examples, the end effector can include further having a flex circuit coupled to the first surface of the electrode landing region and having an electrode metallization layer parallel to the first surface and exposed to ambient environment. The flex circuit can further include being coupled to the at least one extension. The flex circuit can further include having an inductive coil coupled to the at least one extension and curving toward the longitudinal axis parallel to the at least one extension.

In some examples, the end effector can include further having an insulated wire coupled to the flex circuit and in electrical communication with the electrode metallization layer, the insulated wire being positioned facing a second surface of the electrode landing region, the second surface facing toward the longitudinal axis and opposite the first surface.

In some examples, the end effector can further include having an insulated jacket covering a portion of the electrode landing region and providing an opening to expose the electrode. The insulated jacket can include extending across at least a portion of the at least one extension and a second surface of the electrode landing region, the second surface facing toward the longitudinal axis and opposite the first surface. The insulated jacket can also include extending across a majority of the at least one extension and a majority of the second surface of the electrode landing region. The insulate jacket can also include extending over a portion of a flex circuit.

In some examples, the end effector can further include the elongated support member having a substantially uniform thickness over a majority of the length of the end effector.

In some examples, the end effector can further include the elongate support member having a distal extension extending distally from the electrode landing region and a proximal extension extending proximally from the electrode landing region. The electrode landing region can also include having a height measured orthogonal to the longitudinal axis from the first surface to an opposite surface of the at least one extension such that the height is greater than a first thickness of the distal extension and such that the height is greater than a second thickness of the proximal extension.

In some examples, the end effector can also include the elongate support member being formed from a substantially planar sheet cut such that the electrode landing region comprises a first width, a distal extension extending distally from the electrode landing region comprises a second width less than the first width, and a proximal extension extending distally from the electrode landing region comprises a third width less than the first width and approximately equal to the second width.

In some examples, a method of manufacture for an end effector is disclosed. The method can include: cutting a planar sheet to form an elongated support member having an electrode landing region having at least one extension that extends orthogonally from an axis of the elongated support member; curving the at least one extension such that a central portion of the electrode landing region comprises a first planar surface and a second planar surface opposite the first planar surface and such the at least one extension curves away from the first planar surface; and coupling an electrode to the first planar surface.

In some examples, the method can further include coupling an electrode to at least one of the at least one extension.

In some examples, the method can further include curving the at least one extension to overlap at least a portion of the second planar surface.

In some examples, the method can further include forming a recess with the at least one extension, the recess being across a portion of the second planar surface or approximate the second planar surface. The method can also include positioning a portion of an inductive coil within the recess.

In some examples, the method can further include positioning an inductive coil approximate the second planar surface and curving the at least one extension over the inductive coil. In some examples, the axis of the inductive coil can include being parallel to a longitudinal axis of the elongated support member. The axis of the inductive coil can also include being orthogonal to the first surface.

In some examples, wherein the at least one extension can include having a pair of extensions extending symmetrically from a central body of the elongated support member. In some examples, wherein curving the at least one extension can comprise curving each extension of the pair of extensions away from the first planar surface to form symmetrical curves.

In some examples, the method can further include coupling a flex circuit to the first surface of the electrode landing region such that an electrode metallization layer of the flex circuit is parallel to the first surface and exposed to ambient environment. The method can also include coupling the flex circuit to the at least one extension. The method can also include the flex circuit having an inductive coil and coupling the flex circuit to the at least one extension can include positioning the inductive coil across a surface of the at least one extension.

In some examples, the method can further include coupling an insulated wire to the flex circuit.

In some examples, the method can further include positioning an insulated jacket over at least a portion of the electrode landing region.

In some examples, curving the at least one extension can comprise folding the at least one extension at least 180°. Curving the at least one extension can also comprise folding the at least one extension such that an end of the at least on extension approaches the second planar surface.

In some examples, an end effector is disclosed. The end effector can include an elongated support member extending along a longitudinal axis. The end effector can also include an electrode landing pad coupled to the elongated support member such that the electrode landing pad is folded around the elongated support member to provide an outer surface and two side surfaces on opposite sides of the outer surface. The end effector can also include a flex circuit having an electrode metallization layer, the flex circuit being coupled to the outer surface of the electrode landing pad, and the electrode metallization layer being parallel to the outer surface and exposed to ambient environment. The end effector can also include an insulated wire in electrical communication with the electrode metallization layer, the insulated wire being positioned in a recess formed by the elongated support member and/or the electrode landing pad.

In some examples, the end effector can include further having a pair of parallel elongated support members having the elongated support member extending along the longitudinal axis forming a gap between elongated support members of the pair of elongated support members, the gap having the recess in which the insulated wire is positioned.

In some examples, the end effector can also include the recess being positioned approximate an end of the electrode landing pad and approximate an inner surface of the elongated support member.

In some examples, wherein the flex circuit can include being confined to the outer surface of the electrode landing pad.

In some examples, wherein the flex circuit can include extending over at least a portion of at least one of the two side surfaces of the electrode landing pad.

In some examples, the end effector can further include an inductive coil disposed in the recess.

Other aspects and features of the present disclosure will become apparent to those skilled in the pertinent art, upon reviewing the following detailed description in conjunction with the accompanying figures.

### BRIEF DESCRIPTION OF THE DRAWINGS

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation.
FIG. 1 is an illustration of an example catheter-based electrophysiology mapping and ablation system according to aspects of the present invention.
FIG. 2 is an illustration of an end effector according to aspects of the present invention.
FIG. 3A is a cross sectional view illustrating an electrode assembly having an inductive coil disposed within an electrode landing extension and the axis of the inductive coil being parallel to the longitudinal axis according to the aspects of the present invention.
FIG. 3B is a side view illustrating the electrode assembly of FIG. 3A according to aspects of the present invention.
FIGS. 4A is a cross sectional view illustrating an alternate electrode assembly having an inductive coil disposed within an electrode landing extension and the axis of the inductive coil being orthogonal to the first surface according to the aspects of the present invention.
FIG. 4B is a side view illustrating the electrode assembly of FIG. 4A according to aspects of the present invention.
FIG. 5 is a cross-sectional view illustrating an electrode assembly having an insulated wire coupled to an inner surface of an electrode metallization layer of a flex circuit according to aspects of the present invention.
FIG. 6 is a cross-section view illustrating an alternate electrode assembly having an insulated wire coupled to an outer surface of an electrode metallization layer of a flex circuit according to aspects of the present invention.
FIGS. 7A and 7B are illustrations of the dimensions of the elongate support members according to aspects of the present invention.
FIG. 8A is an illustration of alternate dimensions of the elongate support members according to aspects of the present invention.
FIGS. 8B and 8C are illustrations of the dimensions of the elongate support members of the elongate support members of FIG. 8A, wherein the electrode landing extensions are curled around the longitudinal axis according to aspects of the present invention.
FIGS. 9A and 9B are illustrations of an electrode assembly, wherein inductive coils are coupled to the electrode landing extensions according to aspects of the present invention.
FIG. 10A is an illustration of an alternate electrode assembly having an inductive coil and an insulated wire disposed within a recess of an elongated support member according to aspects of the present invention.
FIG. 10B is an illustration of an alternate electrode assembly having an inductive coil and an insulated wire disposed within a recess between an inner contact region of a flex circuit and an elongated support member according to aspects of the present invention.
FIG. 11 is a flow diagram of a method for manufacturing an end effector according to aspects of the present invention.

### DETAILED DESCRIPTION

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

As used herein, the term "microcatheter" is a catheter having a diameter that is small in comparison to catheters in cardiovascular applications, i.e. 8 French or less.

As used herein, the terms "tubular" and "tube" are to be construed broadly and are not limited to a structure that is a right cylinder or strictly circumferential in cross-section or of a uniform cross-section throughout its length. For example, a tubular structure or system is generally illustrated as a substantially right cylindrical structure. However, the tubular system may have a tapered or curved outer surface without departing from the scope of the present disclosure.

Documents incorporated by reference in the present patent application are to be considered an integral part of the application except that to the extent any terms are defined in these incorporated documents in a manner that conflicts with the definitions made explicitly or implicitly in the present specification, only the definitions in the present specification should be considered.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the pertinent art in view of the teachings herein. Such modifications and variations are intended to be included within the scope of the claims.

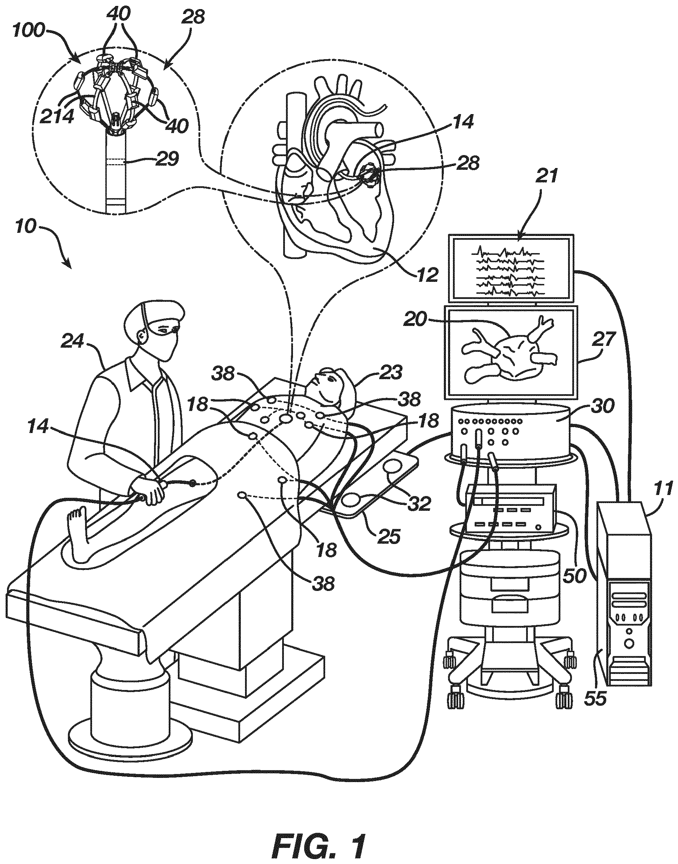

FIG. 1 is an illustration showing an example catheter-based electrophysiology mapping and ablation system 10. The system 10 includes multiple catheters, which are percutaneously inserted by a physician 24 through the patient's vascular system into a chamber or vascular structure of a heart 12. Typically, a delivery sheath catheter is inserted into the left or right atrium near a desired location in the heart 12. Thereafter, a plurality of catheters can be inserted into the delivery sheath catheter so as to arrive at the desired location. The plurality of catheters may include catheters dedicated for sensing Intracardiac Electrogram (IEGM) signals, catheters dedicated for ablating and/or catheters dedicated for both sensing and ablating. An example catheter 14 that is configured for sensing IEGM is illustrated herein. The physician 24 brings a distal tip 28 of the catheter 14 into contact with the heart wall for sensing a target site in the heart 12. For ablation, the physician 24 would similarly bring a distal end of an ablation catheter to a target site for ablating.

The illustrated catheter 14 is an exemplary catheter that includes one and preferably multiple electrodes 26 optionally distributed over a plurality of spines 22 at distal tip 28 and configured to sense the IEGM signals. Catheter 14 may additionally include a position sensor 29 embedded in or near distal tip 28 for tracking position and orientation of distal tip 28. Optionally and preferably, position sensor 29 is a magnetic based position sensor including three magnetic coils for sensing three-dimensional (3D) position and orientation.

A magnetic based position sensor 29 may be operated together with a location pad 25 including a plurality of magnetic coils 32 configured to generate magnetic fields in a predefined working volume. Real time position of a distal tip 28 of the catheter 14 may be tracked based on magnetic fields generated with a location pad 25 and sensed by a magnetic based position sensor 29. Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091 incorporated by reference herein.

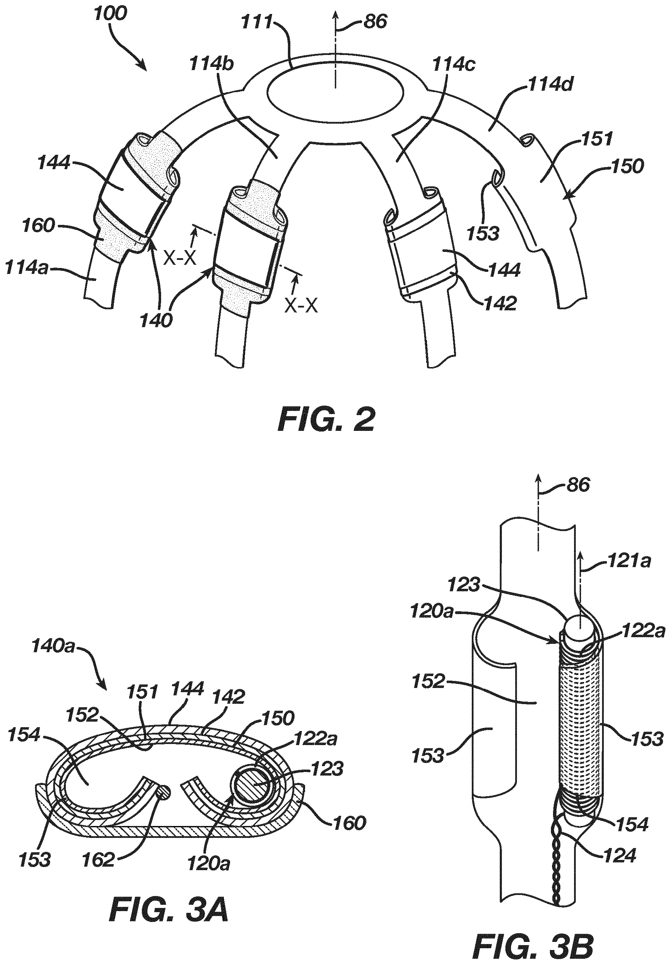

The end effector 100 can include one or more inductive coils 120 coupled to an inner side of the spines 214 under the electrodes 26 as illustrated in Figs. 3A, 3B, 4A, 4B, and Fig. 9A and otherwise disclosed elsewhere herein. These inductive coils can be configured as a single axis magnetic based position sensor. The coils may further be combined to provide three axis sensing. For instance, the end effector 100 can be modified to include compatible features of navigation sensors which include three coil arrangements aligned along three orthogonal axes to determine position and orientation of the navigation sensor in three dimensions disclosed for instance in U.S. Patent Numbers 10,330,742 and 10,677,857, each included by reference as if set forth herein and attached in the Appendix of parent Patent Application No. 63/503,754.

The system 10 includes one or more electrode patches 38 positioned for skin contact on the patient 23 to establish location reference for location pad 25 as well as impedance-based tracking of electrodes 26. For impedance-based tracking, electrical current is directed toward electrodes 26 and sensed at electrode skin patches 38 so that the location of each electrode can be triangulated via the electrode patches 38. Details of the impedance-based location tracking technology are described in US Patent Nos. 7,536,218; 7,756,576; 7,848,787; 7,869,865; and 8,456,182 incorporated by reference herein.

A recorder 11 displays electrograms 21 captured with body surface ECG electrodes 18 and intracardiac electrograms (IEGM) captured with electrodes 26 of the catheter 14. The recorder 11 may include pacing capability for pacing the heart rhythm and/or may be electrically connected to a standalone pacer.

The system 10 can include an ablation energy generator 50 that is adapted to conduct ablative energy to one or more of electrodes at a distal tip of a catheter configured for ablating. Energy produced by the ablation energy generator 50 may include, but is not limited to, radiofrequency (RF) energy or pulsed-field ablation (PFA) energy, including monopolar or bipolar high-voltage DC pulses as may be used to effect irreversible electroporation (IRE), or combinations thereof. The electrodes 26 may be configured to provide RF or IRE signals to tissue to perform ablation. PFA generates substantially less heat than RF and therefore does not require electrodes able to withstand temperatures of electrodes suitable for RF. The electrodes 26 can therefore be made thinner (e.g. flex circuit layer) for PFA applications. For instance, the electrodes 26 can be suitable for applying energy as disclosed in U.S. Patent No. 11,540,877 and U.S. Patent Pub. No. 2021/0169550A1, 2021/0169567A1, 2021/0169568A1, 2021/0196372A1, 2021/0177503A1, and 2021/0186604A1, each of which are incorporated herein.

A patient interface unit (PIU) 30 is an interface configured to establish electrical communication between catheters, electrophysiological equipment, power supply and a workstation 55 for controlling operation of system 10. Electrophysiological equipment of the system 10 may include for example, multiple catheters, a location pad 25, body surface ECG electrodes 18, electrode patches 38, an ablation energy generator 50, and a recorder 11. Optionally and preferably, the PIU 30 includes processing capability for implementing real-time computations of location of the catheters and for performing ECG calculations.

The workstation 55 includes memory, processor unit with memory or storage with appropriate operating software loaded therein, and user interface capability. The workstation 55 can be configured to provide multiple functions, optionally including (1) modeling the endocardial anatomy in three-dimensions (3D) and rendering the model or an anatomical map 20 for display on a display device 27; (2) displaying on the display device 27 activation sequences (or other data) compiled from recorded electrograms 21 in representative visual indicia or imagery superimposed on the rendered anatomical map 20; (3) displaying real-time location and orientation of multiple catheters within the heart chamber; and (4) displaying on the display device 27 sites of interest such as places where ablation energy has been applied. One commercial product embodying elements of the system 10 is available as the CARTO^{™} 3 System, available from Biosense Webster, Inc., 31A Technology Drive, Irvine, CA 92618.

FIG. 2 is an illustration of an end effector 100. The catheter 14 illustrated in FIG. 1 can be modified to include features of an end effector 100 illustrated in FIG. 2. The end effector 100 can include a plurality of elongated support members 114a-d extending along a longitudinal axis 86 and coupled at a distal end 111 and a proximal end 112 of an end effector 100. Each of the plurality of elongate support members 114a-d can include an electrode landing region 150 that includes a first surface 151 facing away from the longitudinal axis 86, at least one extension 153 extending from the first surface 151 and curving toward the longitudinal axis 86, and a second surface 152 facing toward the longitudinal axis 86 and opposite the first surface 151 (depicted in FIGS. 3A-9B). The electrode landing region 150 can also include an electrode assembly 140 disposed thereon.

In some examples, the at least one extension 153 can include a pair of extensions 153 symmetrically extending on either side of the electrode landing region 150.

In some examples, the electrode assembly 140 can include a flex circuit 142 coupled to the electrode landing region 150 and the at least one extension 153 of the electrode landing region 150. The flex circuit can also include an electrode metallization layer 144 parallel to the first surface 151 and exposed to ambient environment. The electrode assembly 140 can also include an insulated jacket 160 covering a portion of the electrode landing region 150, extending over a portion of the flex circuit 142, and providing an opening for the exposed electrode metallization layer 144.

In some examples, the insulated jacket 160 can include extending across at least a portion of the at least one extension 153 and at least a portion of the second surface 152 of the electrode landing region 150.

In some examples, the insulated jacket 160 can include extending across a majority of the at least one extension 153 and a majority of the second surface 152 of the electrode landing region 150.

In some examples, the electrode landing region can include a contiguous outer surface such that the contiguous outer surface includes the first surface 151 and a curved outer surface of each of the at least extension 153 and such that a portion of the curved outer surface of the at least one extension 153 faces the longitudinal axis 86. In some examples, the contiguous outer surface can include a P-shaped or a B-shaped profile.

In some examples, the at least one extension 153 can be folded at least 180°. The at least one extension can also be folded such that an end of the at least one extension 153 approaches the second surface 152 of the electrode landing region 150.

FIGS. 3A and 3B are illustrations of an electrode assembly 140a, similar to electrode assembly 140 described above in FIG. 2. However, in some examples, the at least one extension 153 of electrode assembly 140a can form a pocket 154 across a portion of a second surface 152 of the electrode landing region 150 and include an electrical component. The electrical component can include an inductive coil 120a such that a portion of the inductive coil 120a is positioned within the pocket 154. The inductive coil 120a can include an insulated wire strand 122a wound about an axis 121a of the inductive coil 120a with the inductive coil core 123 at its center. The inductive coil 120a can also include an inductive wire 124a attached proximally to the wire strands 122a of inductive coil 120a.

The magnetic based position sensor 29 is positioned proximal the end effector 100 of the catheter, therefore not providing as accurate a position as possible. In addition, this magnetic based position sensor only provides one axis for which to observe the location of the catheter. The inductive coil 120a disposed within pocket 154 provides an additional axis of orientation for which the surgeon operating the device can more accurately observe the physical positioning and orientation of the catheter. The inductive coils 120a of each of the plurality of elongated support members 114 provide their own individual axes which contribute to a detailed image of where the catheter is located within the body of the patient.

In some examples, the axis 121a of the inductive coil 120a can be parallel to the longitudinal axis 86.

In some examples, electrode assembly 140a of FIGS. 3A and 3B can include a flex circuit 142 coupled to the electrode landing region 150 and the at least one extension 153 of the electrode landing region 150. The flex circuit can also include an electrode metallization layer 144 parallel to the first surface 151 and exposed to ambient environment. The electrode assembly 140a can also include an insulated jacket 160 covering a portion of the electrode landing region 150, extending over a portion of the flex circuit 142, and providing an opening for the exposed electrode metallization layer 144. The electrode assembly 140a can also include an insulated wire 162 in contact with the exposed electrode metallization layer 144 and parallel with the axis 88 of the elongated support member 114.

In some examples, the at least one extension 153 can further include curving to overlap at least a portion of the second surface 152 of the electrode landing region 150.

In some examples, the elongated support member 114 can comprise a conductive or nonconductive material. Further, the elongated support member 114 can be made from a shape-memory material.

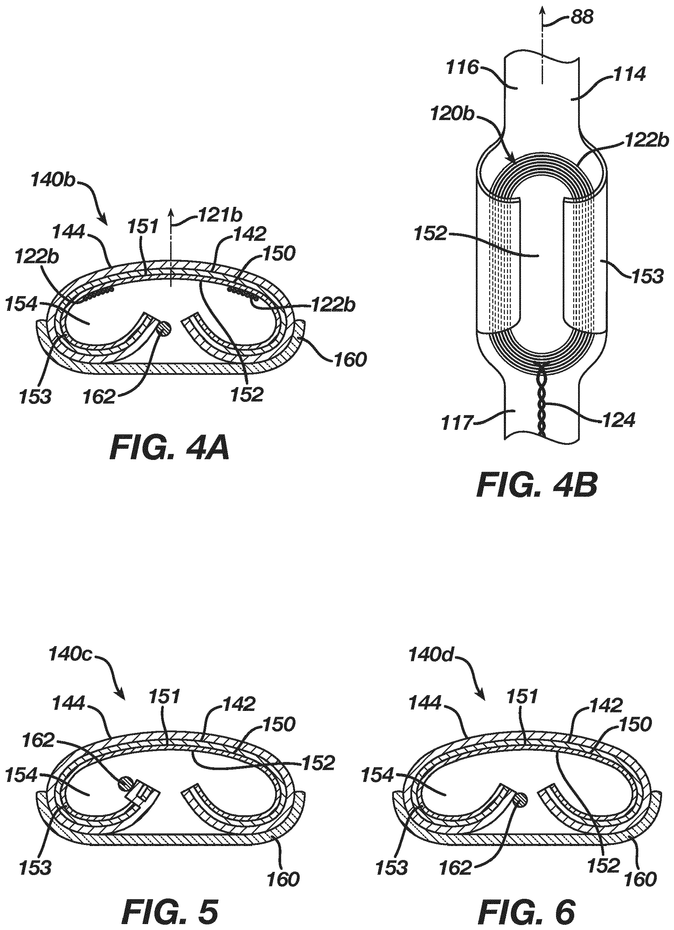

FIGS. 4A and 4B are illustrations of an electrode assembly 140b, similar to electrode assembly 140 described above in FIG. 2. However, in some examples, the at least one extension 153 of electrode assembly 140b can form a pocket 154 across a portion of a second surface 152 of the electrode landing region 150 and include an electrical component. The electrical component can include an inductive coil 120 such that a portion of the inductive coil 120b is positioned within the pocket 154. The inductive coil 120b can include an insulated wire strand 122b wound about an axis 121b of the inductive coil 120b with the inductive coil core 123 at its center. The inductive coil 120b can also include an inductive wire 124b attached proximally to the wire strands 122b of inductive coil 120b.

The magnetic based position sensor 29 is positioned proximal the end effector 100 of the catheter, therefore not providing as accurate a position as possible. In addition, this magnetic based position sensor only provides one axis for which to observe the location of the catheter. The inductive coil 120b disposed within pocket 154 provides an additional axis of orientation for which the surgeon operating the device can more accurately observe the physical positioning and orientation of the catheter. The inductive coils 120b of each of the plurality of elongated support members 114 provide their own individual axes which contribute to a detailed image of where the catheter is located within the body of the patient.

In some examples, the axis 121b of the inductive coil 120b can be orthogonal to the first surface 151.

In some examples, electrode assembly 140b of FIGS. 4A and 4B can include a flex circuit 142 coupled to the electrode landing region 150 and the at least one extension 153 of the electrode landing region 150. The flex circuit can also include an electrode metallization layer 144 parallel to the first surface 151 and exposed to ambient environment. The electrode assembly 140b can also include an insulated jacket 160 covering a portion of the electrode landing region 150, extending over a portion of the flex circuit 142, and providing an opening for the exposed electrode metallization layer 144. The electrode assembly 140b can also include an insulated wire 162 in contact with the exposed electrode metallization layer 144 and parallel with the axis 88 of the elongated support member 114.

FIG. 5 is a cross-section illustration of an electrode assembly 140c, similar to electrode assembly 140 described above in FIG. 2. Electrode assembly 140c can include an electrode landing region 150 that includes a first surface 151, at least one extension 153 extending from the first surface 151 and curving toward a longitudinal axis 86, and a second surface 152 opposite the first surface 151. The electrode assembly 140c can also include a flex circuit 142 coupled to the electrode landing region 150 and the at least one extension 153 of the electrode landing region 150. The flex circuit can also include an electrode metallization layer 144 parallel to the first surface 151 and exposed to ambient environment. The electrode assembly 140 can also include an insulated jacket 160 covering a portion of the electrode landing region 150, extending over a portion of the flex circuit 142, and providing an opening for the exposed electrode metallization layer 144. The at least one extension 153 can include an insulated wire 162 in contact with the electrode metallization layer 144 and facing the second surface 152 of the electrode landing region 150.

In some examples, the insulated wire 162 can also be disposed within the pocket 154 formed by the at least one extension 153 and coupled to the flex circuit 142. The insulated wire 162 is in contact with electrode metallization layer 144 through a via disposed through the flex circuit 142. This allows for the insulated wire 162 to be positioned such that it is still coupled to the flex circuit 142 but in a compact environment with a small surface area. In addition, the positioning of the insulated wire 162 within the pocket 154 also provides security/protection for the connection between the insulated wire 162 and the electrode metallization layer 144. The insulated wire 162 will not impeded any movement when collapsing the end effector 100. The pocket 154 also provides room for slack in the wire in order to provide strain relief for the connection between the insulated wire 162 and the electrode metallization layer 144.

FIG. 6 is a cross-section illustration of an electrode assembly 140d, similar to electrode assembly 140 described above in FIG. 2. Electrode assembly 140d can include an electrode landing region 150 that includes a first surface 151, at least one extension 153 extending from the first surface 151 and curving toward a longitudinal axis 86, and a second surface 152 opposite the first surface 151. The electrode assembly 140d can also include a flex circuit 142 coupled to the electrode landing region 150 and the at least one extension 153 of the electrode landing region 150. The flex circuit can also include an electrode metallization layer 144 parallel to the first surface 151 and exposed to ambient environment. The electrode assembly 140 can also include an insulated jacket 160 covering a portion of the electrode landing region 150, extending over a portion of the flex circuit 142, and providing an opening for the exposed electrode metallization layer 144. The at least one extension 153 can include an insulated wire 162 in contact with the electrode metallization layer 144 and facing the second surface 152 of the electrode landing region 150.

In some examples, the insulated wire 162 can also be disposed outside the pocket 154 formed by the at least one extension 153 and coupled to the flex circuit 142. The insulated wire 162 is in direct contact with the electrode metallization layer 144.

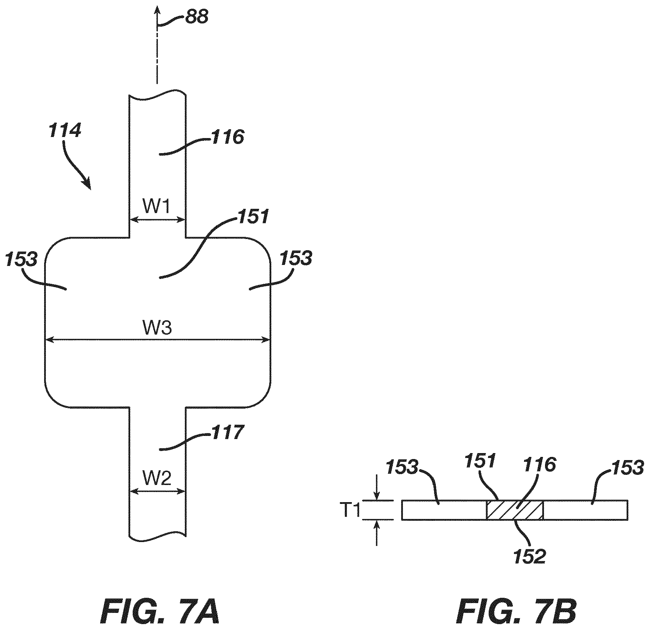

FIGS. 7A and 7B are illustrations of the dimensions of the elongate support member 114 of end effector 100. The elongate support member 114 can include a substantially uniform thickness T1 over a majority of the length of the end effector 100.

In some examples, the elongate support member can include an electrode landing region 150 having a first width W3, a distal extension 116 extending distally from the electrode landing region 150 along the axis 88 of the elongate support member 114 and having a second width W1 less than the first width W3, and a proximal extension 117 extending proximally from the electrode landing region 150 and having a third width W2 less than the first width W3 and approximately equal to the second width W2.

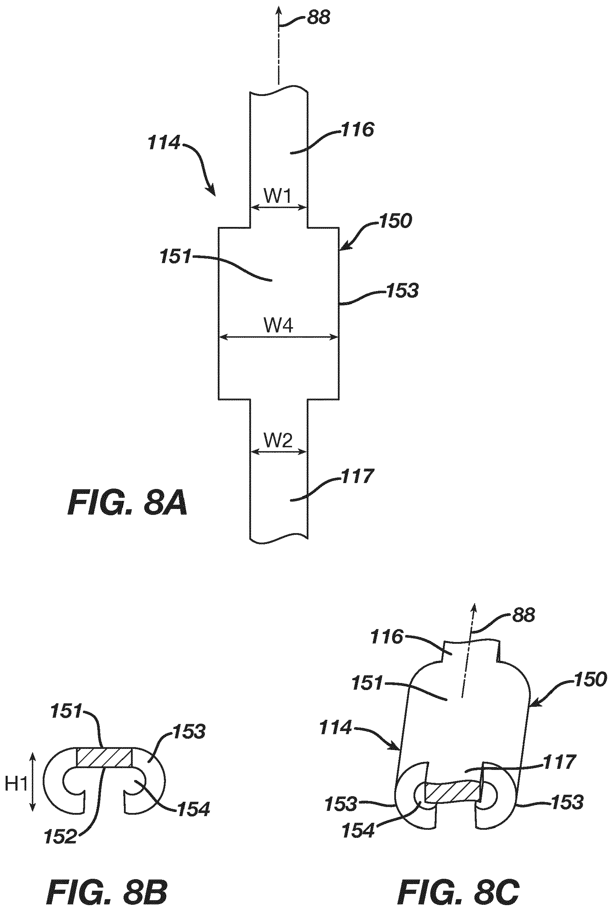

FIGS. 8A-8C are illustrations of the dimensions of the elongate support member 114 of end effector 100. The elongate support member 114 can include a substantially uniform thickness T1 over a majority of the length of the end effector 100.

In some examples, the elongate support member can include an electrode landing region 150 having a first width W4 less than the first width W3 of FIGS. 7A-7B, a distal extension 116 extending distally from the electrode landing region 150 along the axis 88 of the elongate support member 114 and having a second width W1 less than the first width W3, and a proximal extension 117 extending proximally from the electrode landing region 150 and having a third width W2 less than the first width W4 and approximately equal to the second width W2.

In some examples, the electrode landing region 150 can include a height H1 measured orthogonal to the longitudinal axis 88 of the elongate support member 114 such that the height H1 is greater than a thickness T1.

FIGS. 8B and 8C are illustrations of the dimensions of elongate support member 114 wherein the electrode landing extensions are curved toward the longitudinal axis 86 of the end effector 100.

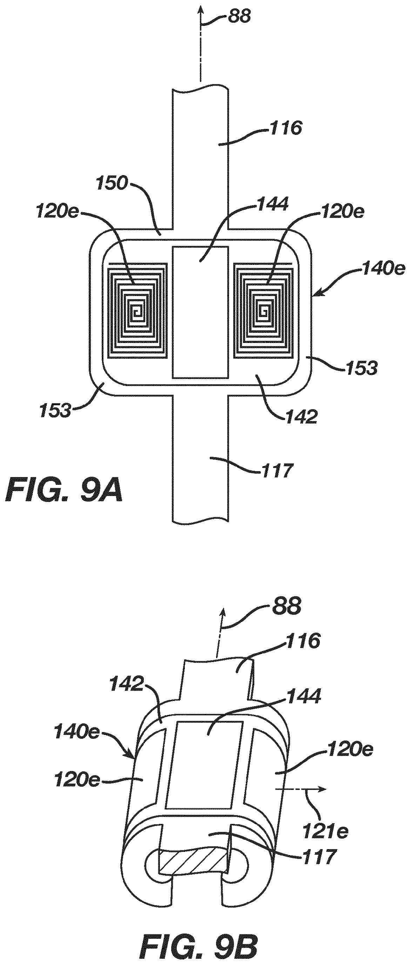

FIGS. 9A and 9B are illustrations of an elongate member that can include an alternate electrode assembly 140e, similar to electrode assembly 140 described in FIG. 2 above. The elongate member can include a distal extension 116, a proximal extension 117, a first surface 151, a second surface 152, and an electrode landing region 150. The electrode landing region 150 can include at least one extension 153 positioned orthogonal to the longitudinal axis 88 of the elongate tubular member 114 and an electrode assembly 140e disposed thereon. The electrode assembly 140e can include a flex circuit 142 coupled to the electrode landing region 150 and the at least one extension 153 of the electrode landing region 150. The flex circuit can also include an electrode metallization layer 144 parallel to the first surface 151 and exposed to ambient environment. The extensions 153 of electrode landing region 150 can include inductive coils 120e disposed thereon such that, when the at least one extension 153 is curved toward the longitudinal axis 86, the axis 121e of the inductive coil 120e is positioned orthogonal to the longitudinal axis 88 of the elongate support member 114 and parallel to the first surface 151.

In some examples, the inductive coils 120e provide an additional axis for which to reference and measure the position of the catheter. This allows for simplified location and orientation of the catheter during operation.

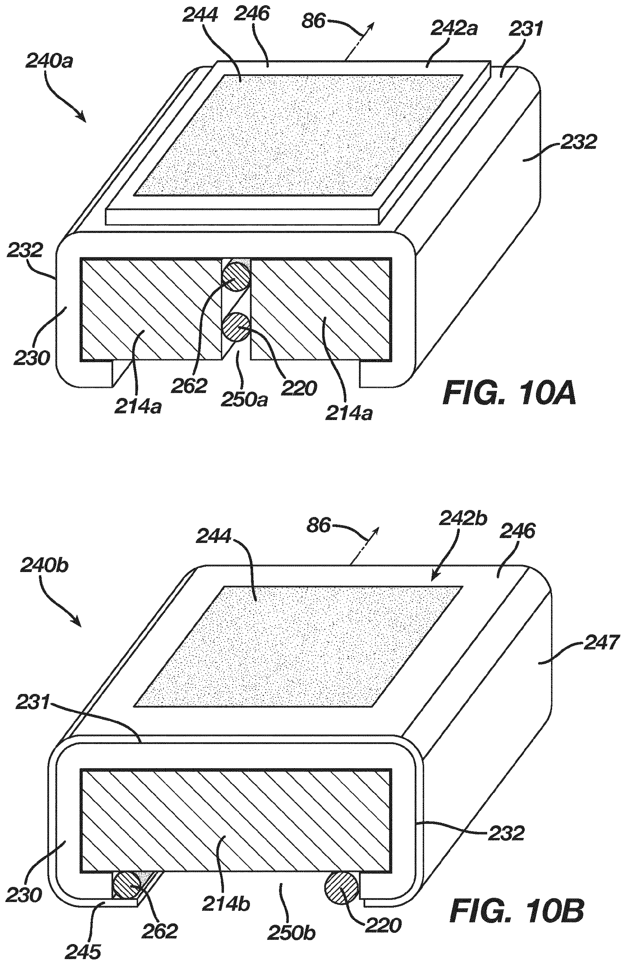

FIG. 10A is an illustration of an alternate electrode assembly 240a. The electrode assembly 240a can include an elongate support member 214a extending along a longitudinal axis 86, an electrode landing pad 230 coupled to the elongate support member 214a, a flex circuit 242a having an electrode metallization layer 244 and its top surface 246 coupled to the outer surface 231 of the electrode landing pad 230, and an insulated wire 262 in electrical communication with the electrode metallization layer 244 and positioned in a gap formed by the elongate support member 214a and/or the electrode landing pad 230. The electrode landing pad can be coupled to the elongate support member 214a such that the electrode landing pad is folded around the elongated support member 214a to provide an outer surface 231 and two side surfaces 232 on opposite sides of the outer surface 231.

In some examples, the electrode metallization layer 244 can be coupled to the outer surface 231 of the electrode landing pad 230 such that it is parallel to the outer surface 231 and exposed to ambient environment.

In some examples, elongate support member 214a can include a pair of parallel elongated support members 214a forming a gap 250a between the pair of elongated support members 214a, the gap 250a having the recess in which the insulated wire 262 is positioned.

In some examples, the gap 250a formed by the pair of elongated support members 214a can also include an inductive coil 220 disposed in the recess.

FIG. 10B is an illustration of an alternate electrode assembly 240b. The electrode assembly 240b can include an elongate support member 214b extending along a longitudinal axis 86, an electrode landing pad 230 coupled to the elongate support member 214b, a flex circuit 242b having an electrode metallization layer 244, and an insulated wire 262 in electrical communication with the electrode metallization layer 244 and positioned in a gap formed by the elongate support member 214b and/or the electrode landing pad 230. The electrode landing pad 230 can be coupled to the elongate support member 214b such that the electrode landing pad 230 is folded around the elongated support member 214b to provide an outer surface 231 and two side surfaces 232 on opposite sides of the outer surface 231.

In some examples, the flex circuit 242b can be disposed over the outer surface 231 of the electrode landing pad. The flex circuit 242b can also extend over at least a portion of at least one of the two side surfaces 232 of the electrode landing pad 230.

In some examples, the electrode metallization layer 244 can be coupled to the outer surface 231 of the electrode landing pad 230 such that it is parallel to the outer surface 231 and exposed to ambient environment.

In some examples, elongate support member 214b can include a gap 250b positioned approximate an end of the electrode landing pad 230 and approximate an inner surface 245 of the elongated support member 214b.

In some examples, the gap 250b can also include an inductive coil 220 disposed within the gap 250b.

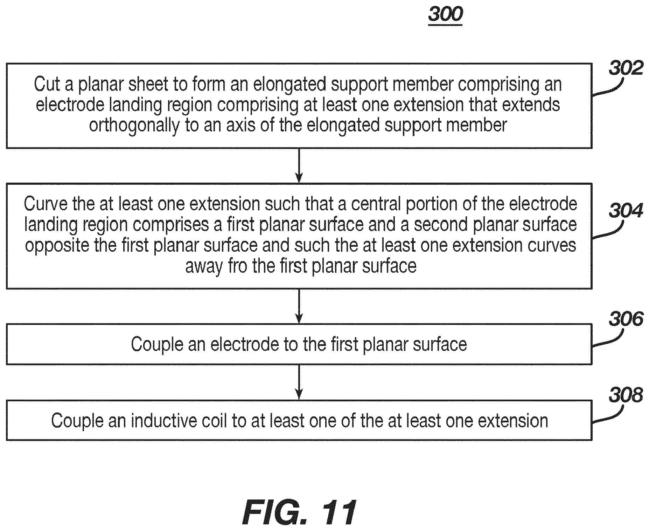

FIG. 11 is a flow diagram of a method 300 for manufacturing an end effector 100 as disclosed herein. The method steps in FIG. 11 can be implemented by any of the example means described herein or by similar means, as will be appreciated. The resulting end effector 100 can be configured similarly to the example end effector 100 presented herein, variations therefore, and alternatives thereto as understood by a person skilled in the pertinent art.

At block 302, the method 300 can include cutting a planar sheet to form an elongated support member 114 having an electrode landing region 150 having at least one extension 153 that extends orthogonally to an axis of the elongated support member 114.

In some examples, the method 300 can further include coupling an electrode 40 to at least one of the at least one extension 153.

At block 304, the method 300 can include curving the at least one extension 153 such that a central portion of the electrode landing region 150 comprises a first planar surface 151 and a second planar surface 152 opposite the first planar surface 151 and such the at least one extension 153 curves away from the first planar surface 151.

In some examples, the at least one extension 153 can include a pair of extensions extending symmetrically from a central body of the elongated support member 114 wherein curving the at least one extension includes curving each extension 153 of the pair of extensions away from the first planar surface 151 to form symmetrical curves.

In some examples, wherein curving the at least one extension 153 can include curving the at least one extension 153 at least 180°.

In some examples, wherein curving the at least one extension 153 can include curving folding the at least one extension 153 such that an end of the at least one extension 153 approaches the second planar surface 152.

In some examples, the method 300 can further include curving the at least one extension 153 to overlap at least a portion of the second planar surface 152.

In some examples, the method 300 can further include forming a recess 154 with the at least one extension 153, the recess 154 being across a portion of the second planar surface 152 or approximate the second planar surface 152. The method 300 can also include positioning a portion of an inductive coil 120 within the recess 154.

In some examples, the method 300 can include positioning the axis 121 of the inductive coil 120 such that the axis 121 is parallel to a longitudinal axis 86 of the elongated support member 114.

In some examples, the method 300 can include positioning the axis 121 of the inductive coil 120 such that the axis 121 is orthogonal to the first planar surface 151.

At block 306, the method 300 can include coupling an electrode 40 to the first planar surface 151.

In some examples, the method 300 can further include coupling a flex circuit 142 to the at least one extension 153 and the first surface 151 of the electrode landing region 150 such that an electrode metallization layer 144 of the flex circuit 142 is parallel to the first surface 151 and exposed to ambient environment. The flex circuit 142 can include an inductive coil 120 positioned across a surface of the at least one extension 153. The flex circuit 142 can also include an insulted wire 162 coupled to the flex circuit 142.

In some examples, the method 300 can further include positioning an insulated jacket 160 over at least a portion of the electrode landing region 150.

At block 308, the method 300 can include coupling an inductive coil 120 to at least one of the at least one extension 153.

The descriptions contained herein are examples of embodiments of the invention and are not intended in any way to limit the scope of the invention. As described herein, the invention contemplates many variations and modifications of an end effector, including alternate insulated wire positioning, alternative means for orientating an inductive coil, etc. Modifications and variations apparent to those having skilled in the pertinent art according to the teachings of this disclosure are intended to be within the scope of the claims which follow.

The following clauses list non-limiting embodiments of the disclosure:
Clause 1. An end effector of a medical probe, the end effector comprising: a plurality of elongated support members extending along a longitudinal axis of the end effector and configured to expand from the longitudinal axis, each of the plurality elongated support members comprising an electrode landing region, the electrode landing region comprising a first surface facing away from the longitudinal axis and at least one extension extending from the first surface and curving toward the longitudinal axis; and an electrode coupled to the first surface.
Clause 2. The end effector of clause 1, further comprising: an inductive coil coupled to the at least one extension.
Clause 3. The end effector of clause 1 or 2, the plurality of elongated support members being coupled to each other at a proximal end of the end effector and at a distal end of the end effector.
Clause 4. The end effector of any one of clauses 1-3, the electrode landing region comprising a contiguous outer surface such that the contiguous outer surface comprises the first surface and a curved outer surface of each extension of the at least one extension and such that at portion of the curved outer surface of each extension of the at least one extension faces the longitudinal axis.
Clause 5. The end effector of clause 4, the contiguous outer surface comprising a P-shaped or B-shaped profile.
Clause 6. The end effector of clause 1, the at least one extension further curving to overlap a portion of a second surface of the electrode landing region, the second surface facing toward the longitudinal axis and opposite the first surface.
Clause 7. The end effector of any one of clauses 1-6, the at least one extension comprising a pair of extensions symmetrically extending on either side of the electrode landing region.
Clause 8. The end effector of any one of clauses 1-7, the at least one extension being folded at least 180°.
Clause 9. The end effector of clause 8, the at least one extension being folded such that an end of the at least on extension approaches a second surface of the electrode landing region, the second surface facing toward the longitudinal axis and opposite the first surface.
Clause 10. The end effector of any one of clauses 1-9, the at least one extension forming a pocket across a portion of a second surface of the electrode landing region, the second surface facing toward the longitudinal axis and opposite the first surface.
Clause 11. The end effector of clause 10, further comprising: an electrical component comprising a portion positioned within the pocket.
Clause 12. The end effector of clause 11, the electrical component comprising an inductive coil such that a portion of the inductive coil is positioned within the pocket.
Clause 13. The end effector of clause 12, the inductive coil comprising an insulated wire strand wound about an axis of the inductive coil.
Clause 14. The end effector of clause 13, the axis of the inductive coil being parallel to the longitudinal axis.
Clause 15. The end effector of clause 13, the axis of the inductive coil being orthogonal to the first surface.
Clause 16. The end effector of any one of clauses 1-15, further comprising: an inductive coil coupled to the at least one extension such that the inductive coil spirals about a sensor axis orthogonal to the longitudinal axis and parallel to the first surface.
Clause 17. The end effector of any one of clauses 1-16, further comprising: a flex circuit coupled to the first surface of the electrode landing region and comprising an electrode metallization layer parallel to the first surface and exposed to ambient environment.
Clause 18. The end effector of clause 17, the flex circuit further being coupled to the at least one extension.
Clause 19. The end effector of clause 18, the flex circuit further comprising an inductive coil coupled to the at least one extension and curving toward the longitudinal axis parallel to the at least one extension.
Clause 20. The end effector of any one of clauses 17-19, further comprising: an insulated wire coupled to the flex circuit and in electrical communication with the electrode metallization layer, the insulated wire being positioned facing a second surface of the electrode landing region, the second surface facing toward the longitudinal axis and opposite the first surface.
Clause 21. The end effector of any one of clauses 1-20, further comprising: an insulated jacket covering a portion of the electrode landing region and providing an opening to expose the electrode.
Clause 22. The end effector of clause 21, the insulated jacket extending across at least a portion of the at least one extension and a second surface of the electrode landing region, the second surface facing toward the longitudinal axis and opposite the first surface.
Clause 23. The end effector of clause 21 or 22, the insulated jacket extending across a majority of the at least one extension and a majority of the second surface of the electrode landing region.
Clause 24. The end effector of any one of clauses 21-23, the insulated jacket extending over a portion of a flex circuit.
Clause 25. The end effector of any one of clauses 1-24, the elongated support member comprising a substantially uniform thickness over a majority of the length of the end effector.
Clause 26. The end effector of any one of clauses 1-25, the elongate support member comprising a distal extension extending distally from the electrode landing region and a proximal extension extending proximally from the electrode landing region, and the electrode landing region comprising a height measured orthogonal to the longitudinal axis from the first surface to an opposite surface of the at least one extension such that the height is greater than a first thickness of the distal extension and such that the height is greater than a second thickness of the proximal extension.
Clause 27. The end effector of any one of clauses 1-26, the elongate support member being formed from a substantially planar sheet cut such that the electrode landing region comprises a first width, a distal extension extending distally from the electrode landing region comprises a second width less than the first width, and a proximal extension extending distally from the electrode landing region comprises a third width less than the first width and approximately equal to the second width.
Clause 28. A method of constructing an end effector, the method comprising: cutting a planar sheet to form an elongated support member comprising an electrode landing region comprising at least one extension that extends orthogonally from an axis of the elongated support member; forming the at least one extension such that a central portion of the electrode landing region comprises a first planar surface and a second planar surface opposite the first planar surface and such the at least one extension curves away from the first planar surface; and coupling an electrode to the first planar surface.
Clause 29. The method of clause 28, further comprising: coupling an electrode to at least one of the at least one extension.
Clause 30. The method of clause 28 or 29, further comprising: curving the at least one extension to overlap at least a portion of the second planar surface.
Clause 31. The method of any one of clauses 28-30, further comprising: forming a recess with the at least one extension, the recess being across a portion of the second planar surface or approximate the second planar surface.
Clause 32. The method of clause 31, further comprising: positioning a portion of an inductive coil within the recess.
Clause 33. The method of any one of clauses 28-32, further comprising: positioning an inductive coil approximate the second planar surface; and curving the at least one extension over the inductive coil.
Clause 34. The method of clause 33, the axis of the inductive coil being parallel to a longitudinal axis of the elongated support member.
Clause 35. The method of clause 33, the axis of the inductive coil being orthogonal to the first surface.
Clause 36. The method of any one of clauses 28-35, wherein the at least one extension comprises a pair of extensions extending symmetrically from a central body of the elongated support member, and wherein curving the at least one extension comprises curving each extension of the pair of extensions away from the first planar surface to form symmetrical curves.
Clause 37. The method of any one of clauses 28-36, further comprising: coupling a flex circuit to the first surface of the electrode landing region such that an electrode metallization layer of the flex circuit is parallel to the first surface and exposed to ambient environment.
Clause 38. The method of clause 37, further comprising: coupling the flex circuit to the at least one extension.
Clause 39. The method of clause 38, wherein the flex circuit comprises an inductive coil, and wherein coupling the flex circuit to the at least one extension positions the inductive coil across a surface of the at least one extension.
Clause 40. The method of any one of clauses 37-39, further comprising: coupling an insulated wire to the flex circuit.
Clause 41. The method of any one of clauses 28-40, further comprising: positioning an insulated jacket over at least a portion of the electrode landing region.
Clause 42. The method of any one of clauses 28-41, wherein curving the at least one extension comprises folding the at least one extension at least 180°.
Clause 43. The method of clause 42, wherein curving the at least one extension comprises folding the at least one extension such that an end of the at least on extension approaches the second planar surface.
Clause 44. An end effector of a medical probe, the end effector comprising: an elongated support member extending along a longitudinal axis; an electrode landing pad coupled to the elongated support member such that the electrode landing pad is folded around the elongated support member to provide an outer surface and two side surfaces on opposite sides of the outer surface; a flex circuit comprising an electrode metallization layer, the flex circuit being coupled to the outer surface of the electrode landing pad, and the electrode metallization layer being parallel to the outer surface and exposed to ambient environment; and an insulated wire in electrical communication with the electrode metallization layer, the insulated wire being positioned in a recess formed by the elongated support member or the electrode landing pad.
Clause 45. The end effector of clause 44. further comprising: a pair of parallel elongated support members comprising the elongated support member extending along the longitudinal axis forming a gap between elongated support members of the pair of elongated support members, the gap comprising the recess in which the insulated wire is positioned.
Clause 46. The end effector of clause 44, the recess being positioned approximate an end of the electrode landing pad and approximate an inner surface of the elongated support member.
Clause 47. The end effector of any one of clauses 44-46, wherein the flex circuit is confined to the outer surface of the electrode landing pad.
Clause 48. The end effector of any one of clauses 44-46, wherein the flex circuit extends over at least a portion of at least one of the two side surfaces of the electrode landing pad.
Clause 49. The end effector of any one of clauses 44-48, further comprising: an inductive coil disposed in the recess.

## Claims

1. An end effector of a medical probe, the end effector comprising:
a plurality of elongated support members extending along a longitudinal axis of the end effector and configured to expand from the longitudinal axis, each of the plurality of elongated support members comprising an electrode landing region, the electrode landing region comprising a first surface facing away from the longitudinal axis and at least one extension extending from the first surface and curving toward the longitudinal axis; and
an electrode coupled to the first surface.

2. The end effector of claim 1, further comprising:
an inductive coil coupled to the at least one extension.

3. The end effector of claim 1 or claim 2, the plurality of elongated support members being coupled to each other at a proximal end of the end effector and at a distal end of the end effector.

4. The end effector of any preceding claim, the electrode landing region comprising a contiguous outer surface such that the contiguous outer surface comprises the first surface and a curved outer surface of each extension of the at least one extension and such that at portion of the curved outer surface of each extension of the at least one extension faces the longitudinal axis, optionally the contiguous outer surface comprising a P-shaped or B-shaped profile.

5. The end effector of claim 1, the at least one extension further curving to overlap a portion of a second surface of the electrode landing region, the second surface facing toward the longitudinal axis and opposite the first surface.

6. The end effector of any preceding claim, the at least one extension comprising a pair of extensions symmetrically extending on either side of the electrode landing region.

7. The end effector of any preceding claim, the at least one extension being folded at least 180° such that an end of the at least on extension approaches a second surface of the electrode landing region, the second surface facing toward the longitudinal axis and opposite the first surface.

8. The end effector of any preceding claim, the at least one extension forming a pocket across a portion of a second surface of the electrode landing region, the second surface facing toward the longitudinal axis and opposite the first surface, optionally further comprising An electrical component comprising a portion positioned within the pocket, further optionally the electrical component comprising an inductive coil such that a portion of the inductive coil is positioned within the pocket, still further optionally the inductive coil comprising an insulated wire strand wound about an axis of the inductive coil.

9. The end effector of any preceding claim, further comprising:
an inductive coil coupled to the at least one extension such that the inductive coil spirals about a sensor axis orthogonal to the longitudinal axis and parallel to the first surface.

10. The end effector of any preceding claim, further comprising:
a flex circuit coupled to the first surface of the electrode landing region and comprising an electrode metallization layer parallel to the first surface and exposed to ambient environment.

11. The end effector of claim 10, the flex circuit further being coupled to the at least one extension and comprising an inductive coil coupled to the at least one extension and curving toward the longitudinal axis parallel to the at least one extension.

12. The end effector of claim 10 or claim 11, further comprising:
an insulated wire coupled to the flex circuit and in electrical communication with the electrode metallization layer, the insulated wire being positioned facing a second surface of the electrode landing region, the second surface facing toward the longitudinal axis and opposite the first surface.

13. The end effector of any preceding claim, further comprising:
an insulated jacket covering a portion of the electrode landing region and providing an opening to expose the electrode and extending over a portion of a flex circuit.

14. The end effector of any preceding claim, each of the plurality of elongated support members comprising a substantially uniform thickness over a majority of the length of the end effector.

15. The end effector of any preceding claim,
each of the plurality of elongated support members comprising a distal extension extending distally from the electrode landing region and a proximal extension extending proximally from the electrode landing region, and
the electrode landing region comprising a height measured orthogonal to the longitudinal axis from the first surface to an opposite surface of the at least one extension such that the height is greater than a first thickness of the distal extension and such that the height is greater than a second thickness of the proximal extension.

16. The end effector of any preceding claim, each of the plurality of elongate support members being formed from a substantially planar sheet cut such that the electrode landing region comprises a first width, a distal extension extending distally from the electrode landing region comprises a second width less than the first width, and a proximal extension extending distally from the electrode landing region comprises a third width less than the first width and approximately equal to the second width.
